# EUROPÄISCHE PATENTSCHRIFT

(11) **EP 2 845 583 B1**
(45) Veröffentlichungstag und Bekanntmachung des Hinweises auf die Patenterteilung: **09.11.2016**
(21) Anmeldenummer: 13182968.1
(22) Anmeldetag: 04.09.2013
(51) Int. Cl.: A61K 8/25, A61K 8/44, A61K 8/67, A61Q 19/08, A61K 8/19

(54) **Kosmetisches Liftingmittel**
Cosmetic lifting agent
Produit de lifting cosmétique

(43) Veröffentlichungstag der Anmeldung: 11.03.2015
(73) Patentinhaber: PM-International AG, 1618 Luxembourg (LU)
(72) Erfinder: Golz-Berner, Karin, MC-9800 Monaco (MC); Meinhardt, Horst, DE-56249 Herschbach (DE); Sorg, Rolf, L-5444 Schengen (LU)
(74) Vertreter: Gulde & Partner

(56) Entgegenhaltungen:
- EP-A2- 0 244 859
- FR-A1- 2 779 059
- US-A1- 2003 059 450
- US-B1- 6 471 972

## Beschreibung

Die Erfindung betrifft ein kosmetisches Mittel mit Lifting-Effekt.

Es sind bereits eine Vielzahl von kosmetischen Mitteln zur Reduzierung von Hautfalten bekannt. In allen Fällen kann nur eine sehr kurzfristige Faltenreduzierung erreicht werden, d. h. es wird für 1 - 2 Stunden ein optischer Effekt erzielt, der dann wieder nachläßt oder es muß eine länger dauernde tägliche Anwendung für einen längeren Zeitraum erfolgen. Im Vordergrund stehen dabei verschiedene Pflanzenextrakte, Enzyme oder auch Peptide (z. B. DE 102009027024 A1). Auch Proteinhydrolysate aus Soja oder Weizen (z. B. WO 2010/049457 A2) werden oft hinzugezogen.

EP-A-0 244 859 offenbart Antifaltenmittel, die Natriumsilicat als anorganischen Antifaltenwirkstoff enthalten. US-A-2003/0059450 schlägt vor, dass zur Faltenreduzierung ein pH-Wert im Bereich von 7,5-13, insbesondere 8,5-11 vorteilhaft ist. US-B-6 471 972 offenbart zudem den Einsatz von Magnesiumaspartat zur Antifaltenbehandlung, insbesondere zur Steigerung der Bindung der Keratinozyten. Außerdem schlägt FR-A-2 779 059 Magnesiumaspartat zur Steigerung der Spannkraft und Reduzierung der Faltentiefe vor.

Aufgabe der Erfindung ist es, einen Anti-Falten- oder Lifting-Effekt bei einmaliger Anwendung für einen längeren Zeitraum zu erreichen und dafür ein Mittel bereitzustellen, das diesen Effekt im wesentlichen mit Hilfe anorganischer Substanzen erreicht. Erfindungsgemäß umfaßt das kosmetische Liftingmittel auf wäßriger Basis ein Gemisch aus Natriumsilicat, dem Magnesiumsalz der Asparaginsäure und aus Ascorbinsäure und hat einen pH-Wert im Bereich von 8,8 bis 9,6.

Der Gehalt an Natriumsilicat (INCI: Sodium Silicate) liegt im Bereich von 12 bis 36 Gew.-%, vorzugsweise 15 bis 22 Gew.-%. Das Natriumsilicat hat vorteilhaft einen acidimetrischen SiO₂-Gehalt im Bereich von 25,5 - 28,5 und einen acidimetrischen Na₂O-Gehalt von 7,5 - 8,5.

Der Gehalt an dem Magnesiumsalz der Asparaginsäure (INCI:Magnesium L-Aspartate) liegt im Bereich von 2,5 bis 8 Gew.-%, insbesondere 3,5 bis 5,5 Gew.-%.

Der Gehalt an Ascorbinsäure, die hier in erster Linie als pH-Regulator dient, liegt im Bereich von 0,5 bis 2 Gew.-%, insbesondere 0,7 bis 1,8 Gew.-%.

Die Konzentrationen sind jeweils auf das Gesamtgewicht des Mittels bezogen.

In einer bevorzugten Ausführungsform der Erfindung umfaßt das kosmetische Liftingmittel auch Antioxidationsmittel, ausgewählt unter Grüntee-Extrakt, Granatapfelextrakt, Rooibus-Extrakt und Gemischen davon. Besonders bevorzugt ist ein Gemisch aus diesen drei Extrakten zu gleichen Teilen, dessen Gehalt im Bereich von 0,15 bis 2,1 Gew.-% liegt, bezogen auf das Gesamtgewicht des Liftingmittels, insbesondere 0,3 bis 1,5 Gew.-%.

Grüntee-Extrakt (INCI: Camellia Sinensis Leaf Extract) und Rooibus-Extrakt (INCI: Aspalathus Linearis Leaf Extract) sind jeweils Blattextrakte mit einem einwertigen Alkohol wie Ethanol. Granatapfelextrakt (INCI: Punica Granatum Fruit Extrakt) ist ein Fruchtextrakt mit einem einwertigen Alkohol wie Ethanol.

Das erfindungsgemäße Mittel kann zusätzlich Solubilisierungsmittel aus Polyolen, wie z. B. Glycerin oder Sorbit, mit Phospholipiden enthalten so beispielsweise solche, die unter der Bezeichnung PhytoSolve® von Lipoid vertrieben werden. Bevorzugt sind dabei PhytoSolve® 4071 und PhytoSolve® 9940. Deren Gehalt liegt insgesamt im Bereich von 0,1 bis 0,5 Gew.-%.

Das erfindungsgemäß eingesetzte Natriumsilicat wird kosmetisch üblicherweise meist nur in geringen Mengen als Penetrationshilfsmittel oder Puffermittel verwendet.

Die Asparaginsäure wird kosmetisch üblicherweise ebenfalls nur in geringen Konzentrationen und insbesondere bei Haarkosmetika als Konditionierungsmittel, in Hautpflegemitteln oder Bräunungsmitteln verwendet.

Der pH-Wert des Mittels liegt vorzugsweise im Bereich von 9,1 - 9,5, insbesondere 9,2 - 9,5.

Die erfindungsgemäße Suspension trennt sich bei längerem Stehenlassen in zwei Schichten auf und ist daher als Schüttelsuspension anzusehen, d. h. vor Verwendung muß der Anwender/die Anwenderin die Suspension kurz aufschütteln und kann diese dann wie eine übliche Lotion auf die Haut auftragen.

Die in der Suspension enthaltenen kleinen Feststoffteilchen mit einem Durchmesser von d₉₀ im Bereich von 1 bis 15 µm werden beim einmaligen Auftragen der Suspension auf der Haut in den Hautfalten abgelagert und füllen diese auf. Die Besonderheit des vorliegenden Mittels liegt darin, daß eine gewisse Bindung an die Oberfläche der Hautfalten erfolgt, und selbst bei einer flüchtigen Benetzung oder Wäsche der Haut mit Wasser werden die Feststoffteilchen nicht oder nur in geringem Maße abgewaschen.

Dadurch ergibt sich schon bei einmaliger Anwendung ein Langzeiteffekt über mehrere Stunden, vorzugsweise 4 - 7 Stunden, der für übliche Antifaltenmittel auf Basis von Pflanzenextrakten oder Peptiden erst über einen längeren Anwendungszeitraum zu erreichen ist.

In jedem Falle wird ein solcher langanhaltender Effekt z. B. durch die enthaltenen Antioxidationsmittel Grüntee-Extrakt, Granatapfelextrakt, Rooibus-Extrakt und deren Gemische keinesfalls erreicht. Dies wurde durch Versuche festgestellt.

Der Teilchendurchmesser d₉₀ bedeutet, daß 90 % der Teilchen in diesem Bereich liegen.

Das erfindungsgemäße Liftingmittel kann noch weitere kosmetische Hilfs- und Zusatzstoffe umfassen.

Dazu gehören kosmetische Öle, insbesondere natürliche Öle auf Pflanzenbasis wie Jojobaöl, Olivenöl, Palmkernöl, Sojabohnenöl, Sonnenblumensamenöl, Weizenkeimöl, Traubenkernöl, Calendulaöl, Avocadoöl und Rapssamenöl.

Es können auch kosmetische Ester und Ether eingesetzt werden, was jedoch nicht bevorzugt ist.

Falls Öle verwendet werden, sind geeignete Emulgatoren erforderlich, insbesondere kationische oberflächenaktive Mittel oder Gemische von anionischen und amphoteren oberflächenaktiven Mitteln wie Plantaren® oder Rewoteric®.

Es können weiterhin einwertige und/oder mehrwertige Alkohole hinzugesetzt werden, wie Ethanol, Glycerin, Propylenglycol, Butylenglycol oder Sorbitol. Der Anteil an Ethanol liegt dann beispielsweise bei 0,3 - 1 Gew.-% und der von Propylenglycol oder einem anderen mehrwertigen Alkohol bei 0,8 - 2,5 Gew.-%.

Die Erfindung betrifft auch Verfahren zur Anwendung eines kosmetischen Liftingmittels auf wäßriger Basis, umfassend ein Gemisch aus Natriumsilicat, dem Magnesiumsalz von Asparaginsäure und aus Ascorbinsäure, das einen pH-Wert im Bereich von 8,8 bis 9,6 hat, wobei das Mittel nach Schütteln bis zur Homogenität als Antifaltenmittel auf entsprechende Hautbereiche wie Gesicht, Hände, Arme, Dekolleté in einer Menge von 0,5 - 2 mg pro cm² aufgetragen wird.

Der langanhaltende Effekt wird noch deutlich weiter verlängert bis zu 20 - 24 Stunden, wenn nach dem Auftragen des Liftingmittels auf die Haut nach einer Pause von 30 - 120 Minuten sehr dünn eine Creme oder ein Make-up auf die gleichen Stellen aufgetragen wird. "Sehr dünn" heißt etwa zwischen 0,5 bis 1,5 mg/cm² Hautfläche.

Die Erfindung soll nachstehend durch Beispiele näher erläutert werden. Alle Mengenangaben sind Gewichtsprozente.

**Beispiel 1 Lifting I**

| | |
|---|---|
| Water | ad 100 % |
| Sodium Silicate (50 %) | 17 |
| Magnesium L-Aspartate | 4,0 |
| PhytoSolve® 9940 | 0,2 |
| Ascorbic Acid | 1,2 |

Die ersten drei Bestandteile werden miteinander verrührt. Dann wird unter starkem Rühren die gebildete Kristallstruktur aufgebrochen und homogenisiert. Anschließend werden die restlichen drei Bestandteile eingerührt, und abschließend wird homogenisiert.

**Beispiel 2 Lifting II**

| | |
|---|---|
| Water | ad 100 % |
| Sodium Silicate (50 %) | 22 |
| Magnesium L-Aspartate | 5,2 |
| PhytoSolve® 9940 | 0,3 |
| PhytoSolve® 4071 | 0,1 |
| Ascorbic Acid | 1,8 |

Es wird wie im Beispiel I verfahren.

**Beispiel 3 Lifting III**

| Water | ad 100 % |
|---|---|
| Sodium Silicate (50 %) | 19 |
| Magnesium L-Aspartate | 4,8 |
| PhytoSolve® 9940 | 0,3 |
| Ascorbic Acid | 1,4 |
| Green Tea Extract (Camellia Sinensis Leaf Extract) | 0,1 |
| Granatapfelextrakt (Punica Granatum Fruit Extract) | 0,1 |
| Rooibosextrakt (Aspalathus Linearis Leaf Extract) | 0,1 |

Es wird wie im Beispiel I verfahren. Die Pflanzenextrakte werden zum Schluß vor dem Homogenisieren eingerührt.

### Beispiel 4 Lifting IV

Die Zusammensetzung entspricht der von Beispiel 3 mit Ausnahme der drei Pflanzenextrakte, die mit jeweils 0,2 Gew.-% enthalten sind, und zusätzlich PhytoSolve® 4071, das mit 0,1 Gew.-% enthalten ist.

### Beispiel 5 Vergleichstest

Es wurde ein Consumer-Test durchgeführt mit 15 weiblichen Probandinnen im Alter von 35 bis 55 Jahren. Die Teilnehmerinnen trugen im Abstand von einer Woche einmalig die Liftingsuspension von Beispiel 1, von Beispiel 3 und von einer AntiFaltencreme auf Gesicht und Dekolleté auf.

Ein Paneel von drei erfahrenen Testern bewertete den optischen Zustand im Abstand von 1 - 3 - 5 - 7 Stunden. Zusätzlich erfolgte eine Bewertung nach 12 Stunden, wenn 1 Stunde nach dem Auftragen der Lifting-Mittel eine einfache Tagescreme dünn (etwa 1 mg/cm²) über die zu bewertenden Flächen aufgebracht worden war.

**Ergebnisse siehe Tabelle (Mittelwerte).**

| Faltenreduzierung in % | | | | | |
|---|---|---|---|---|---|
| | nach 1h | nach 3h | nach 5h | nach 7h | nach 12h |
| Beispiel 1 | 42 | 35 | 28 | 23 | - |
| Beispiel 3 | 44 | 39 | 28 | 25 | - |
| Bsp. 1 + Abdeckung mit Tagescreme | 42 | 39 | 33 | 29 | 22 |
| Antifaltencreme* | 5 | 3 | - | - | - |
| Pflanzencreme** | 7 | 3 | - | - | - |

| | | | | | |
|---|---|---|---|---|---|
| * Die Creme enthielt 0,5 % Retinol und 1,0 % Retinylacetat in einer üblichen Cremegrundlage. ** Die Creme enthielt in üblicher Cremegrundlage jeweils 0,1 % Grüntee-Extrakt, Granatapfelextrakt und Rooibus-Extrakt. | | | | | |

Die Ergebnisse zeigen die Überlegenheit des erfindungsgemäßen Liftingmittels bei einmaliger Anwendung. Im Zeitraum 3 bis 7 Stunden wird noch immer ein deutlicher Faltenreduzierungswert erzielt und damit ein langanhaltender Effekt gegenüber der Vergleichscreme.

## Patentansprüche

1. Kosmetisches Liftingmittel auf wäßriger Basis, **dadurch gekennzeichnet, daß** es ein Gemisch aus Natriumsilicat, aus dem Magnesiumsalz von Asparaginsäure und aus Ascorbinsäure umfaßt und einen pH-Wert im Bereich von 8,8 bis 9,6 hat.

2. Liftingmittel nach Anspruch 1, worin der Gehalt an Natriumsilicat im Bereich von 12 bis 36 Gew.-% liegt, vorzugsweise 15 bis 22 Gew.-%.

3. Liftingmittel nach Anspruch 1 oder 2, worin der Gehalt an dem Magnesiumsalz der Asparaginsäure im Bereich von 2,5 bis 8 Gew.-% liegt, insbesondere 3,5 bis 5,5 Gew.-%.

4. Liftingmittel nach einem der Ansprüche 1 bis 3, worin der Gehalt an Ascorbinsäure im Bereich von 0,5 bis 2 Gew.-% liegt, insbesondere 0,7 bis 1,8 Gew.-%.

5. Liftingmittel nach einem der Ansprüche 1 bis 4, worin zusätzliche Pflanzenextrakte enthalten sind, ausgewählt unter Grüntee-Extrakt, Granatapfelextrakt, Rooibus-Extrakt und Gemischen davon.

6. Liftingmittel nach Anspruch 5, worin der Gehalt des Gemisches der drei Extrakte im Bereich von 0,15 bis 2,1 Gew.-% liegt, insbesondere 0,3 bis 1,5 Gew.-%.

7. Liftingmittel nach einem der Ansprüche 1 bis 6, worin zusätzlich ein oder mehrere Solubilisierungsmittel aus Polyolen mit Phospholipiden enthalten sind.

8. Verfahren zur Anwendung eines kosmetischen Liftingmittels auf wäßriger Basis, umfassend ein Gemisch aus Natriumsilicat, dem Magnesiumsalz von Asparaginsäure und aus Ascorbinsäure, das einen pH-Wert im Bereich von 8,8 bis 9,6 hat, wobei das Mittel nach Schütteln bis zur Homogenität als Antifaltenmittel auf entsprechende Hautbereiche wie Gesicht, Hände, Arme, Dekolleté in einer Menge von 0,5 - 2 mg pro cm² aufgetragen wird.

9. Verfahren nach Anspruch 8, worin im Zeitraum von 30 bis 120 Minuten nach dem Auftragen des Liftingmittels zusätzlich auf die behandelten Hautbereiche eine Tagescreme dünn aufgetragen wird.

## Claims

1. An aqueous-based cosmetic lifting agent, **characterized in that** it comprises a mixture of sodium silicate, the magnesium salt of aspartic acid and ascorbic acid and has a pH value in the range of 8.8 to 9.6.

2. The lifting agent according to Claim 1, wherein the content of sodium silicate is in the range of 12 to 36% by weight, preferably 15 to 22% by weight.

3. The lifting agent according to Claim 1 or 2, wherein the content of the magnesium salt of aspartic acid is in the range of 2.5 to 8% by weight, in particular 3.5 to 5.5% by weight.

4. The lifting agent according to any one of Claims 1 to 3, wherein the content of ascorbic acid is in the range of 0.5 to 2% by weight, in particular 0.7 to 1.8% by weight.

5. The lifting agent according to any one of Claims 1 to 4, wherein it contains additional plant extracts, selected from green tea extract, pomegranate extract, rooibos extract and mixtures thereof.

6. The lifting agent according to Claim 5, wherein the content of the mixture of the three extracts is in the range of 0.15 to 2.1% by weight, in particular 0.3 to 1.5% by weight.

7. The lifting agent according to any one of Claims 1 to 6, wherein it additionally contains one or more solubilizers consisting of polyols with phospholipids.

8. A method for applying an aqueous-based cosmetic lifting agent, comprising a mixture of sodium silicate, the magnesium salt of aspartic acid and ascorbic acid, which has a pH value in the range of 8.8 to 9.6, wherein the agent is applied, following shaking, as an anti-wrinkle agent on appropriate areas of the skin such as the face, hands, arms and décolletage in a quantity of 0.5 - 2 mg per cm² until an homogeneous effect is obtained.

9. The method according to Claim 8, wherein a day cream is, in addition, thinly applied to the treated skin areas within a period of time of 30 to 120 minutes following the application of the lifting agent.

## Revendications

1. Produit de lifting cosmétique à base aqueuse, **caractérisé en ce que** ledit produit comprend un mélange de silicate de sodium, du sel de magnésium d'acide aspartique et d'acide ascorbique, et a une valeur de pH dans une plage de 8,8 à 9,6.

2. Produit de litfing selon la revendication 1, la teneur en silicate de sodium se trouvant dans la plage de 12 à 36 % massique, de préférence de 15 à 22 % massique.

3. Produit de lifting selon la revendication 1 ou 2, la teneur du sel de magnésium de l'acide aspartique se trouvant dans la plage de 2,5 à 8 % massique, en particulier de 3,5 à 5,5 % massique.

4. Produit de lifting selon l'une des revendications 1 à 3, la teneur en acide ascorbique se trouvant dans la plage de 0,5 à 2 % massique, en particulier de 0,7 à 1,8 % massique.

5. Produit de lifting selon l'une des revendications 1 à 4, des extraits de plantes supplémentaires étant contenus, sélectionnés parmi l'extrait de thé vert, l'extrait de grenade, l'extrait de rooibos et des mélanges de ceux-ci.

6. Produit de lifting selon la revendication 5, la teneur en mélange des trois extraits se trouvant dans la plage de 0,15 à 2,1 % massique, en particulier de 0,3 à 1,5 % massique.

7. Produit de lifting selon l'une des revendications 1 à 6, un ou plusieurs moyens de solubilisation à base de polyols avec des phospholipides étant, en outre, contenus.

8. Procédé pour l'utilisation d'un produit de lifting cosmétique à base aqueuse, comprenant un mélange de silicate de sodium, du sel de magnésium de l'acide aspartique et d'acide ascorbique, ayant une valeur de pH dans la plage de 8,8 à 9,6, le moyen étant appliqué après agitation jusqu'à l'homogénéité comme produit antirides sur des zones de la peau correspondantes, telles que le visage, les mains, les bras, la gorge, dans une proportion de 0,5 à 2 mg par cm².

9. Procédé selon la revendication 8, une crème de jour étant de plus appliquée en couche mince sur les zones de la peau traitées, dans un intervalle de 30 à 120 minutes après l'application du produit de lifting.
